Europäisches Patentamt

European Patent Office

Office européen des brevets

(11) Publication number: **0 077 548**
**B1**

(12) EUROPEAN PATENT SPECIFICATION

(45) Date of publication of patent specification: **18.01.89**

(21) Application number: **82109562.7**

(22) Date of filing: **15.10.82**

(51) Int. Cl.⁴: **C 12 N 15/00,** C 12 R 1/15,
C 12 P 13/08, C 12 P 13/14

(54) Plasmid.

(30) Priority: **16.10.81 JP 165511/81**

(43) Date of publication of application:
**27.04.83 Bulletin 83/17**

(45) Publication of the grant of the patent:
**18.01.89 Bulletin 89/03**

(84) Designated Contracting States:
**DE FR GB**

(56) References cited:
**EP-A-0 066 129**
**EP-A-0 071 023**
**FR-A-2 482 622**
**GB-A-2 076 853**

(73) Proprietor: AJINOMOTO CO., INC.
5-8, Kyobashi 1-chome, Chuo-ku
Tokyo 104 (JP)

(72) Inventor: Miwa, Kiyoshi
3-304 Sagamidai-Jutaku, No. 531
Iwase Matsudo-shi Chiba-ken (JP)
Inventor: Terabe, Mahito
No. 1-14, Utsukushigaoka Midori-ku
Yokohama-shi Kanagawa-ken (JP)
Inventor: Tsuchida, Takayasu
No. 1730-13, Kamikurata-cho Totsuka-ku
Yokohama-shi Kanagawa-ken (JP)
Inventor: Ishida, Masaaki
No. 2-20-8, Kannon Kawasaki-ku
Kawasaki-shi Kanagawa-ken (JP)
Inventor: Nakamori, Shigeru
No. 2153-187, Kamariya-cho Kanazawa-ku
Yokohama-shi Kanagawa-ken (JP)
Inventor: Sano, Konosuke
No. 1-35-9, Uehara
Shibuya-ku Tokyo (JP)
Inventor: Momose, Haruo
No. 1968, Pinecrest Drive
East Lansing Michigan 48823 (US)

Courier Press, Leamington Spa, England.

**EP 0 077 548 B1**

(74) Representative: Schübel-Hopf, Ursula et al
Strehl Schübel-Hopf Groening Schulz
Patentanwälte Maximilianstrasse 54 Postfach 22
14 55
D-8000 München 22 (DE)

# EP 0 077 548 B1

**Description for the Contracting States DE GB**

The present invention relates to a purification process for obtaining a substantially pure plasmid, to a substantially pure hybrid plasmid obtained by insertion of a foreign gene into this plasmid and a process for producing a microorganism from Coryneform glutamic acid producing bacteria.

Coryneform glutamic acid producing bacteria belong to so called "Coryneform" bacteria, and are known to produce high amounts of L-glutamic acid, and mutants of the Coryneform glutamic acid-producing bacteria produce amini acid such as lysine and purine nucleotide such as inosinic acid. Therefore. They are of great importance for the fermentation industry. The recently developed gene splicing techniques can successfully be applied for producing or improving industrial microorganisms, especially in case of Escherichia coli. It has been difficult, however, to apply the gene splicing techniques for producing or improving industrial microorganisms of such Coryneform glutamic acid-producing bacteria, since specific plasmids are required for the construction of such industrial microorganisms of Coryneform glutamic acid-producing bacteria.

A plasmid capable of propagating in Coryneform glutamic acid-producing bacteria was reported in Agric. Biol. Chem., 43, 867, (1979) however, the molecular weight of the known plasmid is 37 megadalton and is too large and inconvenient to use for the construction of industrial microorganisms of Coryneform bacteria by gene splicing technique. Therefore, a continuous need existed for the development of a novel plasmid useful for constructing or improving industrial microorganisms from Coryneform glutamic acid-producing bacteria.

EP—A—66 129, a prior art document under Article 54 (3) relates to a method for constructing an L-threonine producing microorganism of the genus Brevibacterium and Corynebacterium, in which a fragment of chromosomal DNA derived from a DNA donor strain is inserted into the plasmid pAM 286 and into the plasmid pAM 330. Hybrid plasmids are obtained from pAM 286 and DNA from Corynebacterium glutamicum NRRL B—15046 and from pAM 330 and DNA from Brevibacterium lactofermentum NRRL B—15048.

In EP—A—71 023, a prior document under Article 54 (3) the construction of L-isoleucin producing microorganism is disclosed, in which the plasmids pAM 286 and pAM 330 are used to obtain hybrid plasmids. Such hybrid plasmids are produced by inserting DNA from Corynebacterium glutamicum NRRL B—15086 into pAM 286 into pAM 286 and a DNA fragment from Brevibacterium lactofermentatum NRRL B—15087 into pAM 330. According to both documents plasmid pAM is obtained from Brevibacterium lactofermentum ATCC 13869, while pAM 286 is obtained from Corynebacterium glutamicum FERM P—5485. The isolation of the plasmids is performed by lysing the cells of the microorganisms, centrifugation of the lysate to obtain a supernatant, concentrating the supernatant and fractionation by means of agarose gel electrophoresis.

FR—A—2 482 622 (published after the priority date of the present application) discloses a hybrid plasmid obtained from pAM 286 and DNA from Corynebacterium glutamicum NRRL B—12 416 and a hybrid plasmid obtained from pAM 330 and DNA from Brevibacterium lactofermentation NRRL B—13 419. Another non-prepublished document, GB—A—2 076 853 relates to hyrid plasmids obtained from pAM 330 and DNA from Brevibacterium lactofermentum NRRL B—12 405 and from pAM 286 and DNA from Corynebacterium glutamicum NRRL B—14 410.

It is the object of the present invention to provide a purification process by which essentially pure plasmids pAM 286 and pAM 330 may be obtained, and to provide novel hybrid plasmids with the use of such purified plasmids, as well as a process for producing a new microorganism.

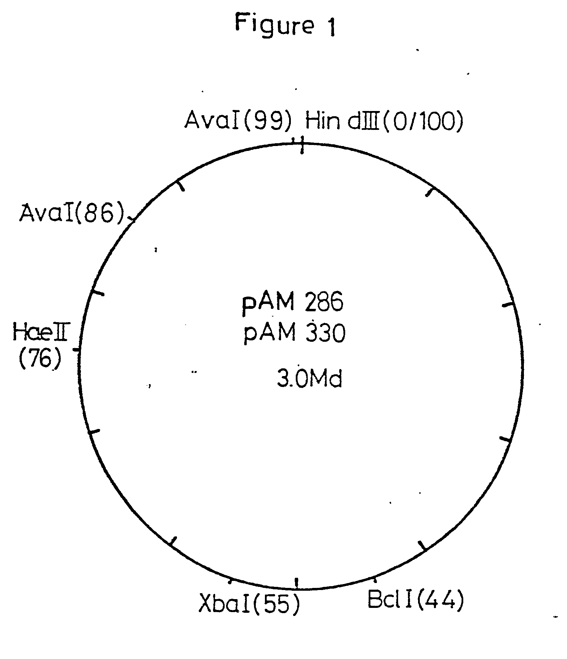

The present invention provides a purification process for obtaining a substantially pure plasmid selected from pAM 330 obtainable from Brevibacterium lactofermentum ATCC 13869 and pAM 286 obtainable from Corynebacterium glutamicum NRRL B—12416 which is characterized by a molecular weight of 3.0 ± 0.1 megadaltons and a restriction endonuclease-cleavage chart shown in Figure 1, which comprises lysing cells of any of the above stated strains previously harvested at the late exponential growth phase by lysozyme and SDS, adding polyethylene glycol to the supernatant obtained from the lysate by centrifugation at 30 000 xg and purifying the precipitated DNA by fractionation by means of cesium chloride-ethidium bromide equilibrium density gradient centrifugation.

The invention further provides a substantially pure hybrid plasmid obtained by insertion of a foreign gene into a plasmid obtainable according to claim 1 having a molecular weight of 3.0 ± 0.1 megadalton and a restriction endonuclease-cleavage chart shown in Figure 1, with the exception of a hybrid plasmid obtained by insertion of DNA from Corynebacterium glutamicum NRRL B—14410, NRRL B—15046 or NRRL B—15086 into pAM 286 and of a hybrid plasmid obtained by insertion of DNA from Brevibacterium lactofermentum NRRL B—14410, NRRL B—15048, or NRRL B—15087 into pAM 330

The invention furthermore provides a process for producing microorganism from Coryneform glutamic producing bacteria, which comprises incorporating into a Coryneform glutamic acid producing bacterium as a recipient a plasmid into wich a gene is inserted, which is responsible for the genetic information for the production of a product, which is characterized in that the above defined hybrid plasmid is incorporated into the recipient.

The pure plasmids obtained have the following common characteristics.

Molecular weights calculated by the migration distance in agarose gel electrophoresis and by the length of the DNA-chain under an electron microscope are both 3.0 megadalton. The sensitivity to restriction enzymes and restriction charts of the two plasmids are the same and shown in Table 1 and Figure 1, respectively.

TABLE 1

| | Restriction Enzyme | Number of Restriction Site |
|---|---|---|
| Alu I | Athrobacter luteus | $\geqq 4$ |
| Ava I | Anabena variabilis | $\geqq 2$ |
| Bcl I | Bacillus caldolyticus | 1 |
| BamH I | Bacillus amyloliquefaciens H | 0 |
| Bgl II | Bacillus globigii | 0 |
| BstE II | Bacillus stearothermophilus ET | $\geqq 4$ |
| EcoR I | Escherichia coli RI+ | 0 |
| Hae II | Haemophilus aegyptius | 1 |
| HgiA I | Herpetosiphon giganteus | $\geqq 4$ |

Brevibacterium lactofermentum ATCC 13869,

Brevibacterium roseum ATCC 13825,

Brevibacterium flavum ATCC 13826,

Brevibacterium thiogenitalis ATCC 19240,

Corynebacterium acetoacidophilum ATCC 13870,

Corynebacterium acetoglutamicum ATCC 15806,

Corynebacterium callunae ATCC 15991,

Corynebacterium glutamicum ATCC 13032, 13060

Corynebacterium lilium ATCC 15990,

Corynebacterium melassecola ATCC

Microbacterium ammoniaphilum ATCC 15354.

Coryneform glutamic acid-producing bacteria also include mutants which have lost the productivity for glutamic acid or have productivity of other amino acids such as lysine and arginine; pure nucleosides such as inosine; purine nucleotides such as inosine-5'-monophosphate; and other fermentation products.

In cells of those Coryneform glutamic acid-producing bacteria, the plasmids according to the present invention are stably maintained. Since these new plasmids can propagate in cells of Coryneform glutamic acid-producing bacteria, the information of a foreign gene inserted in the plasmid can be amplified in the hosts.

The incorporation of plasmid DNA into Coryneform glutamic acid producing bacteria as hosts can be done by treating the cells of the DNA-recipient with calcium chloride to increase the permeability for DNA, as is reported regarding E. coli K—12 (Mandel, M. and Higa, A., J. Mol. Biol., 53, 159 (1970)), or by incorporating at a specific a stage of growth when cells become capable of incorporating DNA (competent cells) as is reported in Bacillus subtilis (Duncan, C. H., Wilson, G. A. and Young, F. E., Gene 1, 153 (1977)).

The plasmid can also be incorporated into the DNA-recipient by forming protoplasts or spheroplasts of the DNA-recipient, which easily incorporate plasmid DNA, as is known in Bacillus subtilis, actinomycetes and yeast (Chang, S. and Choen, S. N., Molec. Gen. Genet., *168,* 111 (1979)); Bibb, M. J., Ward, J. M. and Hopwood, O. A., Nature, *274,* 398 (1978); Hinnen, A., Hicks, J. B. and Fink, G. R., Proc. Nat 1. Acad. Sci., USA. *75,* 1929 (1978)).

A foreign gene can be inserted into the plasmid at the position cleaved by restriction enzymes shown in Table 1 and more preferably at the cleavage position whose number is one. As the foreign gene, genom DNA of Coryneform glutamic acid-producing bacteria is the most preferred. A preferred vector can be obtained from the described plasmid by removing all or a part of DNA regions except the drive-unit (vehicle unit), since the molecular weight becomes small. The replication number of the plasmid is sufficiently large and therefore is suitable for amplifying foreign gene.

## Example 1
### Determination of the replication number and Preparation of pAM 330 DNA

Brevibacterium lactofermentum ATCC 13 869 containing pAM 330 was cultured at 30°C overnight in 5 ml of a minimal medium of pH 7.0 containing per liter, 20 g glucose, 10 g $(NH_4)_2SO_4$, 2.5 g urea, 1 g $KH_2PO_4$, 0.4 g $MgSO_4 \cdot 7H_2O$, 50 µg biotin, 200 µg thiamine · HCl, 0.01 g $FeSO_4 \cdot 7H_2O$ and 0.01 g $MnSO_4 \cdot 4H_2O$, and further added with 100 µCi of $^3H$-thymidine. The cells obtained were lysed by lysozyme and SDS, and circular plasmid was separated by cesium chloride-ethidium bromide equilibrium density gradient centrifugation.

The radioactivity recovery in the circular plasmid was 0.8 to 1.0%. Since the molecular weight of pAM 330 was $3X10^6$ and that of chromosomal DNA of ATCC 13869 was $3X10^9$, the copy number of the circular plasmid was calculated as 8 to 10. (Only circular plasmid can be determined by the method above and plasmid in cell and plasmid which became linear are not included, and therefore, actual copy number may be larger than that determined (Kenji Nagahari; BUNSHI IKUSHU TO OHYOBISEIBUTSU, Kodansha, p172, (1979)).

### Molecular weight of pAM 330

The molecular weight of pAM 330 was calculated from the migration distance on agarose gel electrophoresis and the length of the DNA-chain under an electron microscope. The agarose gel electrophoresis was carried out according to P. A. Sharp's method (Biochemistry *12,* 3055 (1973)), (0.7—0.8% gel, 5 v/cm, and 15 hours). The molecular weight was calculated based on the mobility differences of pBR 322 (Boliver F. et al; Gene, *2,* 95, (1977)), pUB 110 (Gryczan T. J. et al.; J. Bacteriol, *134,* 318, (1978)), and Col $E_1$ (Bazaral M. et al; J. Mol. Biol. *36,* 185, (1968)). The Cytochrome C monolayer method (Kleinschmidt A, Zahn R. K.; Z. Naturforsh, *14b,* 770 (1959)) was applied for the electron microscope observation.

Commercially available restriction enzymes sold by Bethesda Research Laboratory Co., Boehringer Mannheim Co., New England Biolab Co., Worthington Biochemical Co. were used. The digestion by restriction enzyme was carried out using an excessive amount of restriction enzyme, e.g. more than 3 times the amount required. Digestion reaction was carried out according to the well-known directions by the distributors. When the digestion was done by using more than two restriction enzymes, the DNA fragments formed by the last digestion reaction were separated by the method described in T. Tanaka, B. Weisblum; J. Bacteriol., *121,* 354 (1975) and precipitated by ethanol. Thereafter the separated DNA fractions were subjected to the next digestion reaction.

The DNA fragments were thereafter subjected to agarose-gel electrophoresis. The Agarose concentration was 0.7 to 1%, the voltage was 5 to 20 v and the electrophoresis was continued for 1 to 3 hours. The molecular weight was calculated based on the mobility difference between the DNA fragments and authentic samples (φ X 174 RF-Hae III fragment and λ DNA-Hind III fragment both produced and sold by Bethesda Research Labratory).

### Preparation of recombinant DNA from pAM 330

Brevibacterium lactofermentum No. 5116 (NRRL B—12405), a mutant sensitive to a high temperature and induced from strain No. 2256 (ATCC 13869) was cultured at 30°C for 3 hours with shaking in 1l of CMG-medium containing 1g/dl peptone, 1g/dl yeast extract, 0.5g/dl glucose and 0.5g/dl NaCl (pH was adjusted to 7.2), and bacterial cells in the exponential growth phase were harvested. Chromosomal DNA was extracted by a conventional phenol-method, and 3.5mg of purified DNA was obtained.

Ten µg of the chromosomal DNA was treated with each of the restriction endonucleases Hind III at 37°C for 10, 30 and 60 minutes respectively, to cleave DNA chains, and then was heated at 65°C for 5 minutes. Five µg of pAM 330 DNA was also treated with Hind III at 37°C for 1 hour to cleave the DNA completely, and then was heated at 65°C for 5 minutes.

The digested chromosomal DNA solution and the cleaved vector DNA solution were mixed and subjected to the ligation reaction of DNA fragments by a $T_4$ phage DNA-ligase in the presence of ATP and dithiothreitol at 10°C for 24 hours. The reaction mixture was then heated at 65°C for 5 minutes, and the two fold volume of ethanol was added to it. The recombinant DNA which precipitated was recovered.

A glutamic acid requiring strain of Brevibacterium lactofermentum No. 3 (NRRL B—12406) which was derived from Brevibacterium lactofermentum No. 5116 by N-methyl-N'-nitro-N-nitrosoguanidine mutagenesis, was cultured in 20 ml of CMG at 30°C with shaking. Cells in the exponential growth phase were harvested, and suspended in a 0.1M $MgCl_2$ solution and then in a 0.1M $CaCl_2$ solution in an ice-bath, whereby "competent" cells having the ability of DNA uptake were prepared.

Into the competent cell suspension, the recombinant DNA was added. The suspension was kept in an ice-bath for 30 minutes, then heated at 42°C for 2 minutes, and again allowed to stand in an ice-bath for 30 minutes. The cells, thus containing the hybrid plasmid DNA, were inoculated into an L-medium and the medium was shaken at 37°C for 3 hours, whereby the transformation reaction was completed. The cells were harvested, washed, and resuspended. A small portion of the cell suspension was spread on an agar plate containing, 20g glucose, 10g $(NH_4)_2SO_4$, 2.5 g urea, 1g $KH_2PO_4$, 0.4g $MgSO_4 \cdot 7H_2O$, 50 μg biotin, 200 μg thiamine hydrochloride, 0.01g $FeSO_4 \cdot 7H_2O$, 0.01g $MnSO_4 \cdot 4H_2O$ and 20 agar, per liter, (pH was adjusted to 7.2). The plate was incubated at 37°C. After 4 days incubation, all of the colonies which appeared were picked up, purified and isolated.

Strains which became capable of producing L-glutamic acid by the transformation were picked up as the transformants. Among the transformants, most high L-glutamic acid producer AJ 11561 (FERM—P 5469) (NRRL B—12408) was selected. The L-glutamic acid productivity of AJ 11561 was tested comparing with the DNA-donor and the recipients, as follows:

The fermentation medium contained 3.6g/dl glucose, 0.5g/dl urea, 1.0g $KH_2PO_4$, 0.1g/dl $MgSO_4 \cdot 7H_2O$, 3 ml/dl soybean hydrolysate ("Mieki"), 100 μg/l thiamine $\cdot$ HCl 3 μg/1 biotin, 1 mg/dl $FeSO_4 \cdot 7H_2O$, 1 mg/dl $MnSO_4 \cdot 4H_2O$ and 2.5g/dl $CaCO_3$ (separately sterilized) and the pH was adjusted to 7.0.

Twenty ml of the fermentation medium was placed in 500 ml flasks, inoculated with one loopful incoculum of the test microorganisms, and the cultivation was performed at 31°C for 48 hours.

The amounts of L-glutamic acid in the supernatant of the fermentation broth were determined by enzymatic assay.

TABLE 1

| Microorganisms tested | Amounts of L-glutamic acid accumulated (mg/dl) |
|---|---|
| Brevibacterium lactofermentum No. 5116 | 550⁻ |
| Brevibacterium lactofermentum No. 3 | 0 |
| Brevibacterium lactofermentum AJ11561 | 980 |

Example 2

*Preparation of pAM 286 DNA*

Corynebacterium glutamicum AJ 11560 (FERM—P 5485) which contains pAM 286 DNA was cultured in CMG medium at 30°C until the late exponential growth phase. Cells harvested were lysed by lysozyme and SDS treatment. The supernatant of the lysate was separated by centrifugation at 30,000 xg for 30 minutes, and added with polyethyleneglycol to precipitate DNA. The precipitate was dissolved in 1/10 volume of TEN buffer of pH 8.0 containing 20 mM Tris, 20 mM NaCl and 1 mM of EDTA, and thereafter circular plasmid was separated by cesium chloride-ethidium bromide equilibrium density gradient centrifugation. Ethidium bromide was removed from the circular plasmid fraction which was thereafter subjected to dialysis purification, whereby 82 μg of pAM 286 DNA was obtained. The above plasmid DNA isolation procedure was in conformity with the disclosure of Tamio Yamakawa (ed.); SEIKAGAKU JIKKEN KOZA Vol 1, (1) 73, Tokyo Kagaku Donin (1975).

Digestion with restriction enzyme and agarose-gel electro phoresis was performed following the procedure shown in Example 1.

The determination of copy number of pAM 286 was carried out in the manner shown in Example 1 using Corynebacterium glutamicum AJ 11560 as the host, and the copy number was 8 to 10

*Determination of molecular weight of pAM 286*

The molecular weight of pAM 286 was determined by the agarose-gel electrophoresis and electron microscope observation shown in Example 1.

*Preparation of recombinant DNA from pAM 286*

Corynebacterium glutamicum No. 22 (NRRL B—12416), a mutant resistant to S-(2-aminoethyl)-cystein (AEC) and induced from Corynebacterium glutamicum AJ 11560 (FERM—P 5485) (NRRL B—12415), was cultured at 30°C for 3 hours with shaking in 1l of CMG-medium containing 1g/dl peptone, 1g/dl yeast extract, 0.5g/dl glucose and 0.5g/dl NaCl (pH was adjusted to 7.2), and bacterial cells in the exponential growth phase were harvested. Chromosomal DNA was extracted by a conventional phenol-method, and 4.0mg of purified DNA was obtained.

Ten µg of the chromosomal DNA was treated with the restriction endonuclease XbaI at 37°C for 10, 30 or 60 minutes, to cleave DNA chains, and then was heated at 65°C for 5 minutes, respectively. Five µg of the vector DNA was also treated with the restriction endonuclease, XbaI at 37°C for 1 hour to cleave the DNA completely, and then was heated at 65°C for 5 minutes.

The digested chromosomal and vector DNAs were mixed and subjected to the ligation reaction by $T_4$ DNA-ligase in the presence of ATP and dithiothreitol at 10°C for 24 hours. The reaction mixture was then heated at 65°C for 5 minutes, and two fold volumes of ethanol were added to it. The recombinant DNA which precipitated was recovered.

A L-lysine requiring strain, Corynebacterium glutamicum No. 97 (NRRL B—12417), which was derived from Corynebacterium glutamicum No. 22 by N-methyl-N'-nitro-N-nitrosoguanidine mutagenesis, was cultured in 20ml of CMG-medium at 30°C with shaking. Cells in the exponential growth phase were harvested, and "competent" cells having the ability of DNA uptake were prepared by the $CaCl_2$-treatment.

Into the competent cell suspension, the DNA obtained was added, and the DNA was incorporated into the cells. After the transformation reaction, the cell suspension was spread on an agar plate containing, 20g glucose, 10g $(NH_4)_2SO_4$, 2.5g urea, 1g $KH_2PO_4$, 0.4g $MgSO_4 \cdot 7H_2O$, 50 µg biotin, 200 µg thiamine hydrochloride, 0.01g $FeSO_4 \cdot 7H_2O$, 0.01g $MnSO_4 \cdot 4H_2O$, 3.0g AEC $\cdot$ HCl and 20g agar, per liter, (pH was adjusted to 7.0). The plate was incubated at 30°C. After 4 days incubation, all of the colonies, which appeared and got the productivity of L-lysine and resistance to AEC, were picked up, purified and isolated. Thus, AJ 11575 (FERM—P 5501) (NRRL B—12418) was obtained.

The transformants obtained were cultured to test their L-lysine productivity. The DNA-donor strain No. 22 and the recipient strain No. 97 were cultured in the same manner for comparison.

The culture medium contained 10g/dl glucose, 0.5g/dl urea, 4.5g/dl $(NH)_4)_2SO_4$, 0.1g/dl $KH_2PO_4$, 0.04g/dl $MgSO_4 \cdot 7H_2O$, 10mg/dl adenine, 10mg/dl sodium glutamate, 0.1mg/l thiamine $\cdot$ HCl, 0.5mg/l biotin, 1mg/dl $FeSO_4 \cdot 7H_2O$, 10mg/dl $MnSO_4 \cdot 4H_2O$ and 5g/dl $CaCO_3$ (separately sterilized) and the pH was adjusted to 8.0.

Twenty ml batches of the fermentation medium were placed in 500ml flasks, inoculated with one loopful inoculum of the test microorganisms, and the cultivation was performed at 31°C for 70 hours.

The amounts of L-lysine in the supernatant of the fermentation broth were determined by micro-biological assay.

TABLE 1

| Microorganisms tested | Amount of L-lysine accumulated (mg/dl) |
|---|---|
| Corynebacterium glutamicum No. 22 | 120 |
| Corynebacterium glutamicum No. 97 | 12 |
| Corynebacterium glutamicum AJ 11575 | 235 |

In this specification, the term ATCC is used for the deposition at American Type Culture Collection, U.S.A., NRRL for the deposition at Agricultural Research Culture Collection, U.S.A. and FERM for the deposition at Fermentation Research Institute, Agency of Industrial Science and Technology, Ministry of International Trade and Industry, Japan.

**Description for the Contracting State FR**

The present invention relates to a purification process for obtaining a substantially pure plasmid, to a substantially pure hybrid plasmid obtained by insertion of a foreign gene into this plasmid and a process for producing a microorganism from Coryneform glutamic acid and producing bacteria.

Coryneform glutamic acid producing bacteria belong to to so called "Coryneform" bacteria, and are known to produce high amounts of L-glutamic acid, and mutants of the Coryneform glutamic-acid producing bacteria produce amino acid such as lysine and purine nucleotide such as inosinic acid. Therefore, they are of great importance for the fermentation industry. The recently developed gene-splicing techniques can successfully be applied for producing or improving industrial microorganisms, especially in case of Escherichia coli. It has been difficult, however, to apply the gene splicing techniques for producing or improving industrial microorganisms of such Coryneform glutamic acid-producing bacteria, since specific

7

plasmids are required for the construction of such industrial microorganisms of Coryneform glutamic acid-producing bacteria.

A plasmid capable of propagating in Coryneform glutamic acid-producing bacteria was reported in Agric. Biol. Chem., *43*, 867, (1979) however, the molecular weight of the known plasmid is 37 megadalton and is too large and inconvenient to use for the construction of industrial microorganisms of Coryneform bacteria by gene splicing technique. Therefore, a continuous need existed for the development of a novel plasmid useful for constructing or improving industrial microorganisms from Coryneform glutamic acid-producing bacteria.

EP—A—66 129, a prior art document under Article 54 (3) relates to a method for constructing an L-threonine producing microorganism of the genus Brevibacterium and Corynebacterium, in which a fragment of chromosomal DNA derived from a DNA donor strain is inserted into the plasmid pAM 286 and into the plasmid pAM 330. Hybrid plasmids are obtained from pAM 286 and DNA from Corynebacterium glutamicum NRRL B—15046 and from pAM 330 and DNA from Brevibacterium lactofermentum NRRL B—15048.

In EP—A—71 023, a prior document under Article 54 (3) the construction of L-isoleucin producing microorganism is disclosed, in which the plasmids pAM 286 and pAM 330 are used to obtain hybrid plasmids. Such hybrid plasmids are produced by inserting DNA from Corynebacterium glutamicum NRRL B—15086 into pAM 286 and aDNA fragment from Brevibacterium lactofermentum NRRL B—15087 into pAM 330. According to both documents plasmid pAM is obtained from Brevibacterium lactofermentum ATCC 13869, while pAM 286 is obtained from Corynebacterium glutamicum FERM P—5485. The isolation of the plasmids is performed by lysing the cells of the microorganisms, centrifugation of the lysate to obtain a supernatant, concentrating the supernatant and fractionation by means of agarose gel electrophoresis.

plasmid obtained from pAM 286 and DNA from Corynebacterium glutamicum NRRL B—12 416 and a hybrid plasmid obtained from pAM 330 and DNA from Brevibacterium lactofermentum NRRL—B—13 419. Another non-prepublished document, GB—A—2 076 853 relates to hybrid plasmids obtained from pAM 330 and DNA from Brevibacterium lactofermentum NRRL B—12 405 and from pAM 286 and DNA from Corynebacterium glutamicum NRRL B—14 410.

It is the object of the present invention to provide a purification process by which essentially pure plasmids pM 286 and pAM 330 may be obtained, and to provide novel hybrid plasmids with the use of such purified plasmids, as well as a process for producing a new microorganism.

The present invention provides a purification process for obtaining a substantially pure plasmid selected from pAM 330 obtainable from Brevibacterium lactofermentum ATCC 13869 and pAM 286 obtaineable from Corynebacterium glutamicum NRRL B—12416 which is characterized by a molecular weight of 3.0 ± 0.1 megadaltons and a restriction endonuclease-cleavage chart shown in Figure 1, which comprises lysing cells of any of the above stated strains previously harvested at the late exponential growth phase by lysozyme and SDS, adding polyethylene glycol to the supernatant obtained from the lysate by centrifugation at 30 000 xg and purifying the precipitated DNA by fractionation by means of cesium chloride-ethidium bromide equilibrium gradient centrifugation.

The invention further provides a substantially pure hybrid plasmid obtained by insertion of a foreign gene into a plasmid obtainable according to claim 1 having a molecular weight of 3.0 ± 0.1 megadaltons and a restriction endonuclease-cleavage chart shown in Figure 1, with the exception of a hybrid plasmid obtained from pAM 286 and a DNA from Corynebacterium glutamicum NRRL B—12416, NRRL B—14410, NRRL B—115046, NRRL B—15086, and from pAM 330 and a DNA from Brevibacterium lactofermentum NRRL B—12419, NRRL B—12405, NRRL B—15048, NRRL B—15087.

The invention furthermore provides a process for producing a microorganism from Coryneform glutamic acid producing bacteria, which comprises incorporating into a Coryneform glutamic acid producing bacterium as a recipient a plasmid into which a gene is inserted, which is responsible for the genetic information for the production of a product, which is characterized in that the above defined hybrid plasmid is incorporated into the recipient.

The pure plasmids obtained have the following common characteristics.

Molecular weights calculated by the migration distance in agarose gel electrophoresis and by the length of the DNA-chain under an electron microscope are both 3.0 megadalton. The sensitivity to restriction enzymes and restriction charts of the two plasmids are the same and shown in Table 1 and Figure 1, respectively.

TABLE 1

|  | Restriction Enzyme | Number of Restriction Site |
|---|---|---|
| Alu I | Athrobacter luteus | $\geqq 4$ |
| Ava I | Anabena variabilis | $\geqq 2$ |
| Bcl I | Bacillus caldolyticus | 1 |
| BamH I | Bacillus amyloliquefaciens H | 0 |
| Bgl II | Bacillus globigii | 0 |
| BstE II | Bacillus stearothermophilus ET | $\geqq 4$ |
| EcoR I | Escherichia coli RI+ | 0 |
| Hae II | Haemophilus aegyptius | 1 |
| HgiA I | Herpetosiphon giganteus | $\geqq 4$ |

Brevibacterium lactofermentum ATCC 13869,

Brevibacterium roseum ATCC 13825,

Brevibacterium flavum ATCC 13826,

Brevibacterium thiogenitalis ATCC 19240,

Corynebacterium acetoacidophilum ATCC 15870,

Corynebacterium acetoglutamicum ATCC 15806,

Corynebacterium callunae ATCC 15991,

Corynebacterium glutamicum ATCC 13032, 13060

Corynebacterium lilium ATCC 15990,

Corynebacterium melassecola ATCC 17965,

Microbacterium ammoniaphilum ATCC 15354.

Coryneform glutamic acid-producing bacteria also include mutants which have lost the productivity for glutamic acid or have productivity of other amino acids such as lysine and arginine; pure nucleosides such as inosine; purine nucleotides such as inosine-5'-monophosphate; and other fermentation products.

In cells of those Coryneform glutamic acid-producing bacteria, the plasmids obtainable according to the present invention are stably maintained. Since these new plasmids can propagate in cells of Coryneform glutamic acid-producing bacteria, the information of a foreign gene inserted in the plasmid can be amplified in the hosts.

The incorporation of plasmid DNA into Coryneform glutamic acid producing bacteria as hosts can be done by treating the cells of the DNA-recipient with calcium chloride to increase the permeability for DNA, as is reported regarding E. coli K—12 (Mandel, M. and Higa, A., J. Mol. Biol., 53,, 159 (1970)), or by incorporating at a specific a stage of growth when cells become capable of incorporating DNA (competent cells) as is reportred in Bacillus subtilis (Duncan, C. H., Wilson, G. A. and Young, F. E., Gene 1, 153 (1977)).

The plasmid can also be incorporated into the DNA-recipient by forming protoplasts or spheroplasts of the DNA-recipient, which easily incorporate plasmid DNA, as is known in Bacillus subtilis, actinomycetes and yeast (Chang, S. and Choen, S. N., Molec. Gen. Genet., 168, 111 (1979)); Bibb, M. J., Ward, J. M. and Hopwood, O. A., Nature, 274, 398 (1978); Hinnen, A., Hicks, J. B. and Fink, G. R., Proc. Nat 1. Acad. Sci., USA. 75, 1929 (1978)).

A foreign gene can be inserted into the plasmid at the position cleaved by restriction enzymes shown in Table 1 and more preferably at the cleavage position whose number is one. As the foreign gene, genom DNA of Coryneform glutamic acid-producing bacteria is the most preferred. A preferred vector can be obtained from the described plasmid by removing all or a part of DNA regions except the drive-unit (vehicle unit), since the molecular weight becomes small. The replication number of the plasmid is sufficiently large and therefore is suitable for amplifying foreign gene.

Example 1

*Determination of the replication number and Preparation of pAM 330 DNA*

Brevibacterium lactofermentum ATCC 13 869 containing pAM 330 was cultured at 30°C overnight in 5 ml of a minimal medium of pH 7.0 containing per liter, 20 g glucose, 10 g $(NH_4)_2PO_4$, 2.5 g urea, 1 g $KH_2PO_4$, 0.4 g $MgSO_4 \cdot 7H_2O$, 50 µg biotin, 200 µg thiamine $\cdot$ HCl, 0.01 g $FeSO_4 \cdot 7H_2O$ and 0.01 g $MnSO_4 \cdot 4H_2O$, and further added with 100 µCi of $^3H$-thymidine. The cells obtained were lysed by lysozyme and SDS, and circular plasmid was separated by cesium chloride-ethidium bromide equilibrium density gradient centrifugation.

The radioactivity recovery in the circular plasmid was 0.8 to 1.0%. Since the molecular weight of pAM 330 was $3X10^6$ and that of chromosomal DNA of ATCC 13869 was $3X10^9$, the copy number of the circular plasmid was calculated as 8 to 10. (Only circular plasmid can be determined by the method above and plasmid in cell and plasmid which becomes linear are not included, and therefore, actual copy number may be larger than that determined (Kenji Nagahari; BUNSHI IKUSHU TO OHYOBISEIBUTSU, Kodansha, p172, (1979)). $\cdot$

*Molecular weight of pAM 330*

The molecular weight of pAM 330 was calculated from the migration distance on agarose gel electrophoresis and the length of the DNA-chain under an electron microscope. The agarose gel electrophoresis was carried out according to P. A. Sharp's method (Biochemistry *12*, 3055 (1973)), (0.7—0.8% gel, v/cm, and 15 hours). The molecular weight was calculated based on the mobility differences of pBR 322 (Boliver F. et al; Gene, *2*, 95, (1977)), pUB 110 (Gryczan T. J. et al.; J. Bacteriol, *134*, 318, (1978)), and Col $E_1$ (Bazaral M. et al; J. Mol. Biol. *36*, 185, (1968)). The Cytochrome C monolayer method (Kleinschmidt A, Zahn R. K.; Z. Naturforsh, *14b*, 770 (1959)) was applied for the electron microscope observation.

Commercially available restriction enzymes sold by Bethesda Research Laboratory Co., Boehringer Mannheim Co., New England Biolab Co., Worthington Biochemical Co. were used. The digestion by restriction enzyme was carried out using an excessive amount of restriction enzyme, e.g. more than 3 times the amount required. Digestion reaction was carried out according to the well-known directions by the distributors. When the digestion was done by using more than two restriction enzymes, the DNA fragments formed by the last digestion reaction were separated by the method described in T. Tanaka, B. Weisblum; J. Bacteriol., *121*, 354 (1975) and precipitated by ethanol. Thereafter the separated DNA fractions were subjected to the next digestion reaction.

The DNA fragments were thereafter subjected to agarose-gel electrophoresis. The Agarose concentration was 0.7 to 1%, the voltage was 5 to 20 v and the electrophoresis was continued for 1 to 3 hours. The molecular weight was calculated based on the mobility difference between the DNA fragments and authentic samples ($\phi$ X 174 RF-Hae III fragment and λ DNA-Hind III fragment both produced and sold by Bethesda Research Labratory).

*Preparation of recombinant DNA from pAM 330*

Brevibacterium lactofermentum No. 5116, a mutant sensitive to a high temperature and induced from strain No. 2256 (ATCC 13869) was cultured at 30°C for 3 hours with shaking in 1 l, of CMG-medium containing 1g/dl peptone, 1g/dl yeast extract, 0.5g/dl glucose and 0.5g/dl NaCl (pH was adjusted to 7.2), and bacterial cells in the exponential growth phase were harvested. Chromosomal DNA was extracted by a conventional phenol-method, and 3.5mg of purified DNA was obtained.

Ten µg of the chromosomal DNA was treated with each of the restriction endonucleases Hind III at 37°C for 10, 30 and 60 minutes respectively, to cleave DNA chains, and then was heated at 65°C for 5 minutes. Five µg of pAM 330 DNA was also treated with Hind III at 37°C for 1 hour to cleave the DNA completely, and then was heated at 65°C for 5 minutes.

The digested chromosomal DNA solution and the cleaved vector DNA solution were mixed and subjected to the ligation reaction of DNA fragments by a $T_4$ phage DNA-ligase in the presence of ATP and dithiothreitol at 10°C for 24 hours. The reaction mixture was then heated at 65°C for 5 minutes, and the two fold volume of ethanol was added to it. The recombinant DNA which precipitated was recovered.

A glutamic acid requiring strain of Brevibacterium lactofermentum No. 3 (NRRL B—12406) which was derived from Brevibacterium lactofermentum No. 5116 by N-methyl-N'-nitro-N-nitrosoguanidine mutagenesis, was cultured in 20 ml of CMG at 30°C with shaking. Cells in the exponential growth phase were harvested, and suspended in a 0.1M $MgCl_2$ solution and then in a 0.1M $CaCl_2$ solution in an ice-bath, whereby "competent" cells having the ability of DNA uptake were prepared.

Into the competent cell suspension, the recombinant DNA was added. The suspension was kept in an ice-bath for 30 minutes, then heated at 42°C for 2 minutes, and again allowed to stand in an ice-bath for 30 minutes. The cells, thus containing the hybrid plasmid DNA, were inoculated into an L-medium and the medium was shaken at 37°C for 3 hours, whereby the transformation reaction was completed. The cells were harvested, washed, and resuspended. A small portion of the cell suspension was spread on an agar plate containing, 20g glucose, 10g $(NH_4)_2SO_4$, 2.5 g urea, 1g $KH_2PO_4$, 0.4g $MgSO_4 \cdot 7H_2O$, 50 µg biotin, 200 µg thiamine hydrochloride, 0.01g $FeSO_4 \cdot 7H_2O$, 0.01g $MnSO_4 \cdot 4H_2O$ and 20 agar, per liter, (pH was adjusted to 7.2). The plate was incubated at 37°C. After 4 days incubation, all of the colonies which appeared were picked up, purified and isolated.

Strains which became capable of producing L-glutamic acid by the transformation were picked up as the transformants. Among the transformants, most high L-glutamic acid producer AJ 11561 (FERM—P 5469) (NRRL B—12408) was selected. The L-glutamic acid productivity of AJ 11561 was tested comparing with the DNA-donor and the recipients, as follows:

The fermentation medium contained 3.6g/dl glucose, 0.5g/dl urea, 1.0 $KH_2PO_4$, 0.1g/dl $MgSO_4 \cdot 7H_2O$, 3 ml/dl soybean hydrolysate ("Mieki"), 100 µg/l thiamine · HCl 3 µg/l biotin, 1 mg/dl $FeSO_4 \cdot 7H_2O$, 1 mg/dl $MnSO_4 \cdot 4H_2O$ and 2.5g/dl $CaCO_3$ (separately sterilized) and the pH was adjusted to 7.0.

Twenty ml of the fermentation medium was placed in 500 ml flasks, inoculated with one loopful inoculum of the test microorganisms, and the cultivation was performed at 31°C for 48 hours.

The amounts of L-glutamic acid in the supernatant of the fermentation broth were determined by enzymatic assay.

TABLE 1

| Microorganisms tested | Amounts of L-glutamic acid accumulated (mg/dl) |
|---|---|
| Brevibacterium lactofermentum No. 5116 | 550 |
| Brevibacterium lactofermentum No. 3 | 0 |
| Brevibacterium lactofermentum AJ11561 | 980 |

Example 2

*Preparation of pAM 286 DNA*

Corynebacterium glutamicum AJ 11560 (FERM—P 5485) which contains pAM 286 DNA was cultured in CMG medium at 30°C until the late exponential growth phase. Cells harvested were lysed by lysozyme and SDS treatment. The supernatant of the lysate was separated by centrifugation at 30,000 xg for 30 minutes, and added with polyethyleneglycol to precipitate DNA. The precipitate was dissolved in 1/10 volume of TEN buffer of pH 8.0 containing 20 mM Tris, 20 mM NaCl and 1 mM of EDTA, and thereafter circular plasmid was separated by cesium chloride-ethidium bromide equilibrium density gradient centrifugation. Ethidium bromide was removed from the circular plasmid fraction which was thereafter subjected to dialysis purification, whereby 82 µg of pAM 286 DNA was obtained. The above plasmid DNA isolation procedure was in conformity with the disclosure of Tamio Yamakawa (ed.); SEIKAGAKU JIKKEN KOZA Vol 1, (1) 73, Tokyo Kagaku Donin (1975).

Digestion with restriction enzyme and agarose-gel electro phoresis were performed following the procedure shown in Example 1.

The determination of copy number of pAM 286 was carried out in the manner shown in Example 1 using Corynebacterium glutamicum AJ 11560 as the host, and the copy number was 8 to 10.

*Determination of molecular weight of pAM 286*

The molecular weight of pAM 286 was determined by the agarose-gel electrophoresis and electron microscope observation shown in Example 1.

*Preparation of recombinant DNA from pAM 286*

Corynebacterium glutamicum No. 22, a mutant resistant to S-(2-aminoethyl)-cystein (AEC) and induced from Corynebacterium glutamicum AJ 11560 (FERM—P 5485) (NRRL B—12415), was cultured at 30°C for 3 hours with shaking in 1l of CMG-medium containing 1g/dl peptone, 1g/dl yeast extract, 0.5g/dl glucose and 0.5g/dl NaCl (pH was adjusted to 7.2), and bacterial cells in the exponential growth phase were harvested. Chromosomal DNA was extracted by a conventional phenol-method, and 4.0mg of purified DNA was obtained.

Ten µg of the chromosomal DNA was treated with the restriction endonuclease Xbal at 37°C for 10, 30 or 60 minutes, to cleave DNA chains, and then was heated at 65°C for 5 minutes, respectively. Five µg of the vector DNA was also treated with the restriction endonuclease, Xbal at 37°C for 1 hour to cleave the DNA completely, and then was heated at 65°C for 5 minutes.

EP 0 077 548 B1

The digested chromosomal and vector DNAs were mixed and subjected to the ligation reaction by $T_4$ DNA-ligase in the presence of ATP and dithiothreitol at 10°C for 24 hours. The reaction mixture was then heated at 65°C for 5 minutes, and two fold volumes of ethanol were added to it. The recombinant DNA which precipitated was recovered.

A L-lysine requiring strain, Corynebacterium glutamicum No. 97 (NRRL B—12417), which was derived from Corynebacterium glutamicum No. 22 by N-methyl-N'-nitro-N-nitrosoguanidine mutagenesis, was cultured in 20ml of CMG-medium at 30°C with shaking. Cells in the exponential growth phase were harvested, and "competent" cells having the ability of DNA uptake were prepared by the $CaCl_2$-treatment.

Into the competent cell suspension, the DNA obtained was added, and the DNA was incorporated into the cells. After the transformation reaction, the cell suspension was spread on an agar plate containing, 20g glucose, 10g $(NH_4)_2SO_4$, 2.5g urea, 1g $KH_2PO_4$, 0.4g $MgSO_4 \cdot 7H_2O$, 50 µg biotin, 200 µg thiamine hydrochloride, 0.01g $FeSO_4 \cdot 7H_2O$, 0.01g $MnSO_4 \cdot 4H_2O$, 3.0g AEC · HCl and 20g agar, per liter, (pH was adjusted to 7.0). The plate was incubated at 30°C. After 4 days incubation, all of the colonies, which appeared and got the productivity of L-lysine and resistance to AEC, were picked up, purified and isolated. Thus, AJ 11575 (FERM—P 5501) (NRRL B—12418) was obtained.

The transformants obtained were cultured to test their L-lysine productivity. The DNA-donor strain No. 22 and the recipients strain No. 97 were cultured in the same manner for comparison.

The culture medium contained 10g/dl glucose, 0.5g/dl urea, 4.5g/dl $(NH_4)_2SO_4$, 0.1g/dl $KH_2PO_4$, 0.04g/dl $MgSO_4 \cdot 7H_2O$, 10mg/dl adenine, 10mg/dl sodium glutamate, 0.1mg/l thiamine · HCl, 0.5mg/l biotin, 1mg/dl $FeSO_4 \cdot 7H_2O$, 10mg/dl $MnSO_4 \cdot 4H_2O$ and 5g/dl $CaCO_3$ (separately sterilized) and the pH was adjusted to 8.0.

Twenty ml batches of the fermentation medium were placed in 500ml flasks, inoculated with one loopful inoculum of the test microorganisms, and the cultivation was performed at 31°C for 70 hours.

The amounts of L-lysine in the supernatant of the fermentation broth were determined by micro-biological assay.

TABLE 1

| Microorganisms tested | Amount of L-lysine accumulated (mg/dl) |
|---|---|
| Corynebacterium glutamicum No. 22 | 120 |
| Corynebacterium glutamicum No. 97 | 12 |
| Corynebacterium glutamicum AJ 11575 | 235 |

In this specification, the term ATCC is used for the deposition at American Type Culture Collection, U.S.A., NRRL for the deposition at Agricultural Research Culture Collection, U.S.A. and FERM for the deposition at Fermentation Research Institute, Agency of Industrial Science and Technology, Ministry of International Trade and Industry, Japan.

**Claims for the Contracting States DE and GB**

1. A purification process for obtaining a substantially pure plasmid selected from pAM 330 obtainable from Brevibacterium lactofermentum ATCC 13869 and pAM 286 obtainable from Corynebacterium glutamicum NRRL B—12416 which is characterized by a molecular weight of 3.0 ± 0.1 megadaltons and a restriction endonuclease-cleavage chart shown in Figure 1, which comprises lysing cells of any of the above stated strains previously harvested at the late exponential growth phase by lysozyme and SDS, adding polyethylene glycol to the supernatant obtained from the lysate by centrifugation at 30 000 xg and purifying the precipitated DNA by fractionation means of cesium chloride-ethidium bromide equilibrium density gradient centrifugation.

2. A substantially pure hybrid plasmid obtained by insertion of a foreign gene into a plasmid obtainable according to claim 1 having a molecular weight of 3.0 ± 0.1 megadaltons and a restriction endonuclease-cleavage chart shown in Figure 1, with the exception of a hybrid plasmid obtained by insertion of DNA from Corynebacterium glutamicum NRRL B—14410, NRRL B—15046 or NRRL B—15086 into pAM 286 and of a hybrid plasmid obtained by insertion of DNA from Brevibacterium lactofermentum NRRL B—14410, NRRL B—15048 or NRRL B—15087 into pAM 330.

3. A process for producing a microorganism from Coryneform glutamic acid producing bacteria, which comprises incorporating into a Coryneform glutamic acid producing bacterium as a recipient a plasmid into

12

# EP 0 077 548 B1

which a gene is inserted, which is responsible for the genetic information for the production of a product, characterized in that the plasmid according to claim 2 is incorporated into the recipient.

**Claims for the Contracting State FR**

1. A purification process for obtaining a substantially pure plasmid selected from pAM 330 obtainable from Brevibacterium lactofermentum ATCC 13869 and pAM 286 obtainable from Corynebacterium glutamicum NRRL B—12416 which is characterized by a molecular weight of 3.0 ± 0.1 megadaltons and a restriction endonuclease-cleavage chart shown in Figure 1, which comprises lysing cells of any of the above stated strains previously harvested at the late exponential growth phase by lysozyme and SDS, adding polyethylene glycol to the supernatant obtained from the lysate by centrifugation at 30 000 xg and purifying the precipitated DNA by fractionation means of cesium chloride-ethidium bromide equilibrium density gradient centrifugation.

2. A substantially pure hybrid plasmid obtained by insertion of a foreign gene into a plasmid obtainable according to claim 1 having a molecular weight of 3.0 ± 0.1 megadaltons and a restriction endonuclease-cleavage chart shown in Figure 1, with the exception of a hybrid plasmid obtained from pAM 286 and a DNA from Corynebacterium glutamicum NRRL B—12416, NRRL B—14410, NRRL B—15046, NRRL B—15086, and from pAM 330 and a DNA from Brevibacterium lactofermentum NRRL B—12419, NRRL B—12405, NRRL B—15048, NRRL B—15087.

3. A process for producing a microorganism from Coryneform glutamic acid producing bacteria, which comprises incorporating into a Coryneform glutamic acid producing bacterium as a recipient a plasmid into which a gene is inserted, which is responsible for the genetic information for the production of a product, characterized in that the plasmid according to claim 2 is incorporated into the recipient.

**Patentansprüche für die Vertragsstaaten DE und GB**

1. Reinigungsverfahren zum Erzielen eines im wesentlichen reinen Plasmids, das ausgewählt ist unter pAM 330, erhältlich aus Brevibacterium lactofermentum ATCC 13869, und pAM 286, erhältlich aus Corynebacterium glutamicum NRRL B—12416, das gekennzeichnet ist durch ein Molekulargewicht von 3,0 ± 0,1 Megadalton und eine in Figur 1 gezeigte Restriktionsendonuclease-Spaltungskarte, welches die Lyse von Zellen eines der vorstehend genanntnen Stämme, die zuvor in der späten exponentiellen Wachstumsphase geerntet worden sind, mit Lysozym und SDS, die Zugabe von Polyethylenglycol zu dem aus dem Lysat durch Zentrifugieren bei 30 000 xg erhaltenen Überstand und die Reinigung der ausgefällten DNA durch Fraktionierung mit Hilfe der Cäsiumchlorid-Ethidium-bromid-Gleichgewichtsgradienten-Zentrifugation umfaßt.

2. Im wesentlichen reines Hybridplasmid, erhalten durch Insertion eines fremden Gens in ein gemäß Anspruch 1 erhältliches Plasmid mit einem Molekulargewicht 3,0 ± 0,1 Megadalton und einer in Figur 1 gezeigten Restriktionsendonuclease-Spaltungskarte, mit der Ausnahme eines Hybridplasmids, das durch Insertion von DNA aus Corynebacterium glutamicum NRRL B—14410, NRRL B—15046 oder NRRL B—15086 in pAM 286 erhalten wurde une eines Hybridplasmids, das durch Insertion von DNA aus Brevibacterium lactofermentum NRRL B—14410, NRRL B—15048 oder NRRL B—15087 in pAM 330 erhalten wurde.

3. Verfahren zur Herstellung eines Mikrooganismus aus coryneformen Glutaminsäure bildenden Bakterien, bei dem einem coryneformen Glutaminsäure bildenden Bakterium als Empfängerorganismus ein Plasmid einverleibt wird, in welches ein Gen insertiert wurde, welches für die genetische Information für die Bildung eines Produkts verantwortlich ist, dadurch gekennzeichnet, daß das Plasmid gemäß Anspruch 2 dem Empfängerorganismus einverleibt wird.

**Patentansprüche für den Vertragsstaat FR**

1. Reinigungsverfahren zum Erzielen eines im wesentlichen reinen Plasmids, das ausgewählt ist unter pAM 330, erhältlich aus Brevibacterium lactofermentum ATCC 13869, und pAM 286, erhältlich aus Corynebacterium glutamicum NRRL B—12416, das gekennzeichnet ist durch ein Molekulargewicht von 3,0 ± 0,1 Megadalton und eine in Figur 1 gezeigte Restriktionsendonuclease-Spaltungskarte, welches die Lyse von Zellen eines der vorstehend genanntnen Stämme, die zuvor in der späten exponentiellen Wachstumsphase geerntet worden sind, mit Lysozym und SDS, die Zugabe von Polyethylenglycol zu dem aus dem Lysat durch Zentrifugieren bei 30 000 xg erhaltenen Überstand und die Reinigung der ausgefällten DNA durch Fraktionierung mit Hilfe der Cäsiumchlorid-Ethidium-bromid-Gleichgewichtsgradienten-Zentrifugation umfaßt.

2. Im wesentlichen reines Hybridplasmid, erhalten durch Insertion eines fremden Gens in ein gemäß Anspruch 1 erhältliches Plasmid, das eine Molekulargewicht von 3,0 ± 0,1 Megadalton und eine in Figur 1 gezeigte Restriktionsendonuclease-Spaltungskarte, mit der Ausnahme eines Hybridplasmids, erhalten aus pAM 286 und einer DNA aus Corynebacterium glutamicum NRRL B—12416, NRRL B—14410, NRRL B—15046, NRRL B—15086, und aus pAM 330 und einer DNA aus Brevibacterium lactofertum NRRL B—12419, NRRL B—12405, NRRL B—15048, NRRL B—15087.

13

3. Verfahren zur Herstellung eines Mikroorganismus aus coryneformen Glutaminsäure bildenden Bakterien, bei dem einem coryneformen Glutaminsäure bildenden Bakterium als Empfängerorganismus ein Plasmid einverleibt wird, in welches ein Gen insertiert wurde, welches für die genetische Information für die Bildung eines Produkts verantwortlich ist, dadurch gekennzeichnet, daß das Plasmid gemäß Anspruch 2 dem Empfängerorganismus einverleibt wird.

**Revendications pour les Etats Contractants: DE GB**

1. Procédé de purification pour obtenir un plasmide essentiellement pur choisi parmi pAM 330 pouvant être obtenu à partir de Brevibacterium lactofermentum ATTC 13869 et pAM 286 pouvant être obtenu à partir de Corynebacterium glutamicum NRRL B—12416, qui est caractérisé par un poids moléculaire de 3,0 ± 0,1 mégadaltons et la carte de clivage par les endonucléases de restriction illustrée par la figure 1, qui comprend la lyse des cellules de l'une quelconque des souches mentionées ci-dessus, préalablement récoltées dans la phase de croissance exponentielle tardive, avec du lysozyme et du dodécylsulfate de sodium, l'addition de polyéthyléneglycol au surnageant obtenu à partir du lysat par centrifugation à 30 000 × g et la purification de l'ADN précipité par fractionnement par centrifugation avec un gradient de densité de chlorure de césium-bromure d'éthidium en équilibre.

2. Plasmide hybride essentiellement pur obtenu par insertion d'un gène étranger dans un plasmide pouvant être obtenu selon la revendication 1, ayant un poids moléculaire de 3.0 ± 0.1 mégadaltons et la carte de clivage par les endonucléases de restriction illustrée par la figure 1, à l'exception d'un plasmide hybride obtenu par insertion d'un ADN de Corynebacterium glutamicum NRRL B—14410, NRRL B—15046 ou NRRL B—15086 dans pAM 286 et d'un plasmide hybride obtenu par insertion d'un ADN de Brevibacterium lactofermentum NRRL B—14410, NRRL B—15048 ou NRRL B—15087 dans pAM 330.

3. Procédé pour produire un micro-organisme à partir de bactéries Corynéformes productrices d'acide glutamique, qui comprend l'incorporation à une bactérie Corynéforme productrice d'acide glutamique servant de receveur d'un plasmide dans lequel est inséré un gène responsable de l'information génétique pour la production d'un produit, caractérisé en ce que le plasmide selon la revendication 2 est incorporé au receveur.

**Revendications pour l'Etat contractant: FR**

1. Procédé de purification pour obtenir un plasmide essentiellement pur choisi parmi pAM 330 pouvant être obtenu à partir de Brevibacterium lactofermentum ATTC 13869 et pAM 286 pouvant être obtenu à partir de Corynebacterium glutamicum NRRL B—12416, qui est caractérisé par un poids moléculaire de 3,0 ± 0,1 mégadaltons et la carte de clivage par les endonucléases de restriction illustrée par la figure 1, qui comprend la lyse des cellules de l'une quelconque des souches mentionnées ci-dessus, préalablement récoltées dans la phase de croissance exponentielle tardive, avec due lysozyme et du dodécylsulfate de sodium, l'addition de polyéthylèneglycol au surnageant obtenu à partir du lysat par centrifugation à 30 000 × g et la purification de l'ADN précipité par fractionnement par centrifugation avec un gradient de densité de chlorure de césium-bromure d'éthidium en équilibre.

2. Plasmide hybride essentiellement pur obtenu par insertion d'un gène étranger dans un plasmide pouvant être obtenu selon la revendication 1, ayant un poids moléculaire de 3,0 ± 0,1 mégadaltons et la carte de clivage par les endonucléases de restriction illustrée par la figure 1, à l'exception d'un plasmide hybride obtenu à partir de pAM 286 et d'un ADN de Corynebacterium glutamicum NRRL B—12416, NRRL B—14410, NRRL B—15046, NRRL B—15086, et à partir de pAM 330 et d'un ADN de Brevibacterium Lactofermentum NRRL B—12419, NRRL B—12405, NRRL B—15048 et NRRL B—15087.

3. Procédé pour produire un micro-organisme à partir de bactéries Corynéformes productrices d'acide glutamique, qui comprend l'incorporation à une bactérie Corynéforme productrice d'acide glutamique servant de receveur d'un plasmide dans lequel est inséré un gène responsable de l'information génétique pour la production d'un produit, caractérisé en ce que le plasmide selon la revendication 2 est incorporé au receveur.

# Figure 1